# EUROPEAN PATENT SPECIFICATION

(11) **EP 0 414 915 B1**
(45) Date of publication and mention of the grant of the patent: **12.06.1996**
(21) Application number: 90903948.9
(22) Date of filing: 06.03.1990
(51) Int. Cl.: C12N 9/02, C12N 15/53, C12N 1/19, C12N 1/21, A61K 38/44

(54) **NEW SUPEROXIDE DISMUTASE**
NEUE SUPEROXYD-DISMUTASE
NOUVELLE SUPEROXYDE DISMUTASE

(30) Priority: 06.03.1989 JP 52141/89
(43) Date of publication of application: 06.03.1991
(73) Proprietor: SUNTORY LIMITED, Kita-ku, Osaka-shi, Osaka 530 (JP); INOUE, Masayasu, Kumamoto-shi Kumamoto 860 (JP)
(72) Inventor: INOUE, Masayasu, Kumamoto 860 (JP); AMACHI, Teruo, Takarazuka-shi Hyogo 665 (JP); TANAKA, Yoshikazu 15-205, 1, Wakayamadai 2-chome, Osaka 618 (JP)
(74) Representative: Stoner, Gerard Patrick
(86) International application number: JP9000286
(87) International publication number: WO9010694

(56) References cited:
- WO-A-89/12677
- JP-A-63 273 473
- PROCEEDINGS OF THE NATL. ACADEMY OF SCIENCES USA, vol. 84, no. 19, October 1987, Washington, DC (US); L. TIBELL et al., pp. 6634-6638
- BIOCHEMICAL JOURNAL, vol. 242, 1987; K. KARLSSON et al., pp. 55-59
- PROCEEDINGS OF THE NATL. ACADEMY OF SCIENCES USA, vol. 84, no. 18, September 1987, Washington, DC (US); K. HJALMARSSON et al., pp. 6340-6344

## Description

### TECHNICAL FIELD

The present invention is related to novel derivatives of superoxide dismutase (hereinafter abbreviated as SOD), and more specifically, to heparin-binding superoxide dismutases (hereinafter abbreviated as HB-SOD), genes for the HB-SOD, a process for the production of HB-SOD, and prophylactic and therapeutic pharmaceuticals comprising HB-SOD as an effective ingredient, for diseases wherein a superoxide directly or indirectly provides an adverse effect on an organism.

### BACKGROUND ART

SOD is an enzyme which destroys harmful superoxides (active oxygen molecules), and is present in all organs and intracellular fractions (cytoplasm and mitchondria). Although the enzyme called extracellular SOD is present in the blood, its activity is very low, and therefore, its defense mechanism in the cell membrane and in the space outside of tissue cells is very weak, and then oxidative disorders highly risky to an organism sometimes occur in these local sites. The intravenous administration and topical application of SOD has been attempted, to alleviate the oxidative tissue destructive disorders, but these means do not exhibit sufficient defensive actions due to an in vivo instability and short half-life (several minutes) thereof.

Recently, various chemical modifications have been made in an attempt to prolong the half-life of SOD in the blood. For example, modifications with polyethylene glycol (Japanese Unexamined Patent Publication (KOKAI) No. 61-249388), with high molecular weight dextran (W.F. Petrone et al., Proc. Natl. Acad. Sci. USA, 77, 1159 (1980)), with inulin (Japanese Unexamined Patent Publication (KOKAI) No. 58-32826) or the like. These modifications, however, have the various drawbacks described below, and therefore, do not provide a satisfactory resolution of the difficultites involved. Moreover, an SOD derivative (SMA-SOD) wherein stylenemaleate butyl ester derivative (SMA) has been bonded to a lysine residue of the enzyme via an amide bond was devised, to form a complex with serum proteins comprising mainly albumin in the blood (Masayasu Inoue et al., Protein Nucleic Acid and Enzyme, 33, 2889 (1988)), but there is a danger that repeated administration of such a non-natural, chemically modified SOD will cause unexpected side effects, such as an immune response.

As described above, although SOD effectively eliminates harmful superoxide radicals, the required pharmacological effects are not always obtained due to a rapid excretion thereof after administration. On the other hand, SODs which have been modified with various chemical modification methods have been developed as a defense against oxidative tissue disorders of an organism in the blood aqueous space. Although some thereof are stable in the blood, it has been trouble as a drug in that its diffusion rate into tissues lowered accompanying a macromolecular formation and an absolute structure of the compound can not be defined due to variety of site and level of chemical modification to SOD. Since modified SODs are modified with agents which are not or rarely present in nature, an administration of modified SODs can give rise to side effects. Moreover, to obtain such modified SODs, a complicated procedure involving a purification of native SOD, a chemical modification, and a further purification becomes necessary.

Therefore, the development of a novel SOD which comprises materials present in vivo, has very little side effects, specifically protects injured tissues, can be produced in an large amount at a low cost, and has an absolute structure determined, is urgently required.

### DISCLOSURE OF THE INVENTION

Accordingly, the present invention provides a novel superoxide dismutase wherein a heparin-binding domain is added to a superoxide dismutase not originally having a heparin-binding domain.

The present invention also provides a DNA coding for the above-mentioned novel superoxide dismutase, and further, provides a host transformed with the DNA.

### BRIEF EXPLANATION OF DRAWING

Figures 1(1) and 1(2) represent processes for the construction of three HB-SOD genes;
Fig. 2A represents a nucleotide sequence of the HB-SOD gene in pBRHBSOD1 and a corresponding amino acid sequence;
Fig. 2B represents a nucleotide sequence of the HB-SOD gene in pMHBSOD2 and a corresponding amino acid sequence;
Fig. 2C represents a nucleotide sequence of the HB-SOD gene in pBRHBSOD3 and a corresponding amino acid sequence;
Figs. 3 and 4(1) represent processes for the construction of yeast expression vectors for HB-SOD, pYHBS1, pYHBS2, pYHBS3, pYHBS11 and pYHBS12;
Fig. 4(2) represents a process for the construction of an E. coli expression plasmid pKHBS2;
Fig. 5 represents an expression of HB-SOD in yeast;
Fig. 6 represents a thermostability of HB-SODs;
Fig. 7 represents an adsorption of HB-SOD to heparin Sepharose;
Fig. 8 summarizes pharmacodynamics of HB-SOD in the blood;
Fig. 9 summarizes the distribution of HB-SOD among organs;
Fig. 10 represents a protective action of HB-SOD against myocardosis due to ischemic reperfusion;
Fig. 11 summarizes a protective effect of HB-SOD against stress stomach ulcer; and
Fig. 12 summarizes a protective effect of HB-SOD against foot edema.

### BEST MODE OF CARRYING OUT THE INVENTION

As a result of an investigation into the above-mentioned problems, the present inventors produced a heparin-binding SOD (HB-SOD) having a heparin-binding peptide at its carboxyl end, by bonding a gene coding for a heparin-binding peptide to a human erythrocyte SOD gene. The HB-SOD is a novel SOD which is very stable in the blood and has a tropism to vascular endothelial cells in tissues, especially the liver. This SOD does not become a macromolecule, and can bind cells of various tissues and destroy superoxides at that location. Therefore, the present HB-SOD has a remarkable effect on a wide range of oxygen disorders such as traumatic brain edema, ischemic myocardosis, stomach ulcer, edema, and ischemic liver disorder, and thus can be used for prophylactic or therapeutic agents for these diseases.

The present HB-SOD is believed to act at the internal wall of a blood vessel, and after being degraded, is converted to SOD as well as a hepalin-binding peptide and degradation products thereof, and the SOD is then rapidly metabolyzed and excreted into the urine. It is known that proteins having a heparin-binding peptide are numerous in the inner wall of blood vessels, and therefore, it is unthinkable that the heparin-binding peptide and degradation products thereof can exhibit side effects.

Where the present HB-SOD is used for the above-mentioned diseases, various formulations and administration methods can be used; for example, HB-SOD may be intravenously injected after being dissolved in a 5% glucose solution or a physiological saline.

The SOD used in the present invention may be any SOD which originally does not have a heparin-binding domain, including those present in organisms such as animals (e.g., human, cattle), plants or microorganisms; by using a modified SOD, the tropism of the SOD can be enhanced. As heparin-binding domains, a heparin-binding domain of an other SOD originally having a heparin-binding domain, such as, a heparin-binding domain of extracellular SOD, and a heparin-binding domain of a polypeptide other than SOD, such as, a heparin binding site of human antithrombin III, can be used. As these SODs or heparin-binding domains, not only those having a native amino acid sequence but also those having the addition, lack, replacement and/or transfer of one or more than one amino acid, can be used in the present invention as long as they maintain their native activities or functions.

The present invention will be now further illustrated by, but is by no means limited to, the following Examples. Note, DNA recombination techniques used in the Examples follow the techniques of Maniatis et al. (T. Maniatis et al., Molecular Cloning, Gold Spring Lab. (1982)) unless otherwise specified. Restriction enzymes used in the DNA recombination experiments were purchased from Toyobo.

### Example 1. Construction of gene for HB-SOD

### (1) Preparation of human RNA and construction of cDNA library

A total RNA was obtained from a human placenta according to a conventional procedure using guanidinium thiocyanate, and using oligo dT cellulose column chromatography, polyadenylated RNAs were obtained as a mRNA fraction from the total RNA. This RNA fraction was used to prepare a double-stranded DNA preparation using a cDNA synthesis kit (Amersham) prepared according to a method of Gubler (Gubler, U. et al., Gene 25, 263, (1983)). To this double-stranded DNA was added deoxy CTP homopolymers using terminal deoxynucleotidyl transferase (Pharmacia), and this was annealed with a vector wherein deoxy GTP homopolymers have been added to a PstI site of pBR322, and E. coli DH1 was transformed to construct a cDNA library consisting of about 400,000 clones.

### (2) Selection of Transformants

First, as a probe for detecting cDNA of human SOD, DNA A039 having the following sequence: 5' GAAAGTACAAAGACAGGAAACGCTGGAAGTCGTTTGGCTTG 3' was synthesized. This sequence is a sequence contained in an already known cDNA nucleotide sequence of human SOD [Sherman et al., Proc. Natl. Acad. Sci. USA, 80, 5465 (1983)]. The synthesis was carried out by using a 381A DNA synthesizer made by Applied Biosystems. This probe was labeled using (γ-³²P) ATP (Amersham) and T4-polynucleotidylkinase, and used as a probe for the detection of the SOD gene. An E. coli transformant was obtained from the above-mentioned cDNA library by colony hybridization using the probe. This transformant was cultured according to a conventional procedure, and plasmid DNA was extracted from the cells. This plasmid contained a 600 bp cDNA of human SOD gene which was excised with PstI, and was designated pSO10. This insert was sequenced by dideoxy method using M13 phage [for example, Takanami et al., Zoku-Seikagaku Jikken Koza, Idenshi Kenkyu Ho II, Tokyo Kagaku Dojin (1986)]. In the determined nucleotide sequence, a gene ACG coding for 54th amino acid (threonine) had been mutated to ACA, and a gene coding for N-terminal three amino acid residues was lacking. This sequence was a sequence contained in an already reported nucleotide sequence (Hallewell et al., Nucl. Acids. Res. 13, 2007 (1985)).

### (3) Construction of HB-SOD gene

To prepare a lacking amino-termical gene and a start codon, as well as an EcoRI site for linking to an expression vector, the following experiment was carried out.

A synthetic DNA A071, i.e., 5' GGGGGGGGGGAATTCATGGCGACGAAGGCCGTGTGC 3' was prepared by using a DNA synthesizer 381A made by Applied Biosystems, as described above, and using this synthetic DNA as a primer, and a recombinant single-stranded DNA obtained by cloning a 600 bp DNA fragment excised from pSO10 with PstI into M13pm19 as a template, a site-directed mutagenesis was carried out according to Zoller's method [M.J. Zoller et al., Nuc. Acid. Res. 10, 6487 (1982)]. The insert of this mutant was sequenced and it was confirmed that a full-length SOD cDNA was obtained, because there existed a gene coding for amino terminal three amino acid residues, a start codon, and an EcoRI site. The recombinant M13 double-stranded DNA having this mutation was prepared, and a 600 bp DNA fragment excised by PstI was subcloned into a PstI site in pUC9. Among the resulting recombinants, a plasmid containing a 600 bp DNA excised by EcoRI and PstI was designated pSO100. A 450 bp DNA fragment excised from the SO100 with EcoRI and Sau3AI was subcloned into EcoRI and BamHI sites of pBR322, and the resulting plasmid was designated pBRSOD1.

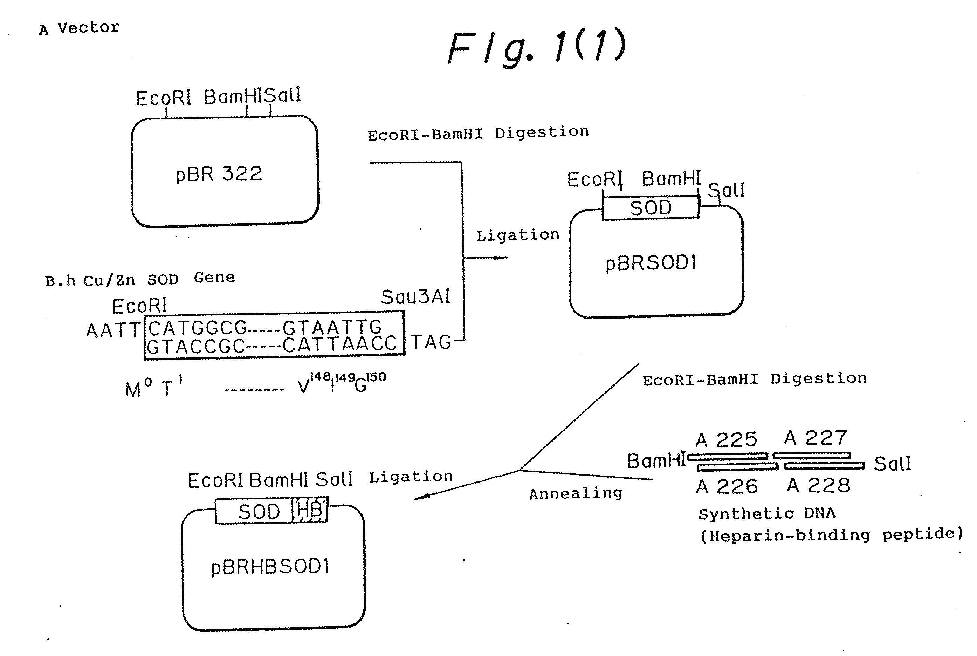

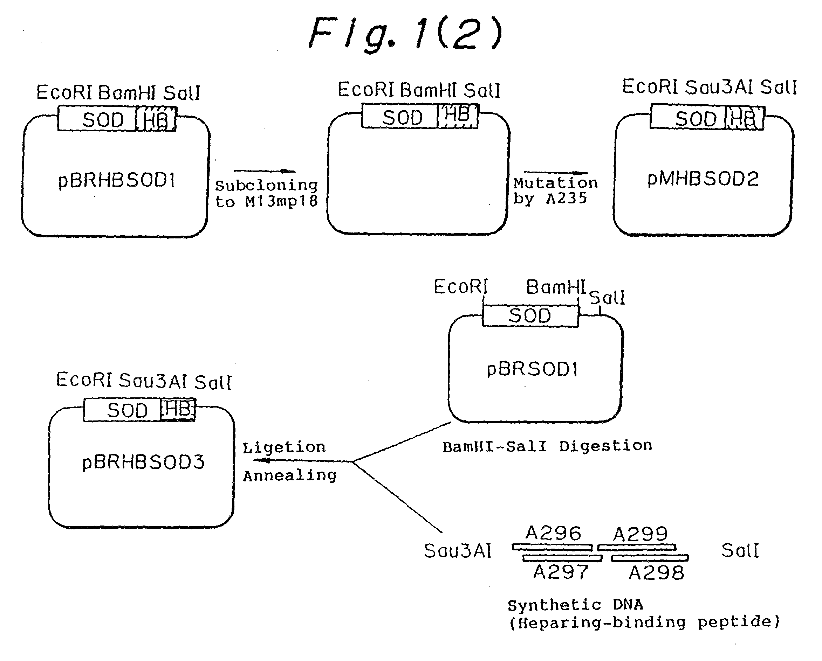

A nucleotide sequence coding for a heparin-binding peptide was synthesized on the basis of a nucleotide sequence for a heparin-binding peptide of extracellular SOD known as an example of heparin-binding peptides (K. Hjalmarsson et al., Proc. Natl. Acad. Sci. USA, 84, 6340, 1987). Namely, 
A225 5′ GATCCGCGGCCCGGGCTCTGGAGCGCCAGGCGC
GGGAGCACT 3′,
 
A226 5′ TCTTGCGCTCTGAGTGCTCCCGCGCCTGGCGCTCC
CAGAGCCCGGGCCCGCG 3′
 
A227 5′ CAGAGCGCAAGAAGCGGCGGCGCGAGAGCGAGTGC
AAGGCCGCCTGAG 3′,
 
A228 5′ TCGACTCAGGCGGCCTTGCACTCGCTCTCGCGCCG
CCGTC 3′
 were synthesized. According to the Ikehara et al method (M. Ikehara et al., Proc. Natl. Acad. Sci., USA, 81, 5956, 1984), these four synthetic DNA were phosphorylated, annealed, and ligated with a DNA fragment of about 4.5 kb obtained by digesting pBRSOD1 with BamHI and SalI, to obtain a plasmid designated pBRHBSOD1. The pBRHBSOD1 had a structure such that a gene for SOD and a heparin-binding peptide joined to each other could be excised with EcoRI and SalI (Fig. 1).

Next, to construct an HB-SOD gene having a different sequence, the DNA fragment excised with EcoRI and SalI was subcloned into EcoRI and SalI sites of M13mp18. Using this single-stranded DNA as a template and a synthetic DNA A235 5' GGGCCCGCAGATCCCAAT 3' as a primer, a site-directed mutagenesis was carried out as described above. A double-stranded DNA having a mutation was designated pMHBSOD2 (Fig. 1). A nucleotide sequence of the DNA was determined, and it was found that the HB-SOD encoded by this plasmid was different from the HB-SOD encoded by pBRHBSOD1, by two amino acid residues.

Moreover, to construct an HB-SOD gene having another sequence, a gene coding for a peptide of a heparin-binding site of human antithrombin III was synthesized. Although the structure followed the procedures of Chandra et al. [T. Chandra et al. Proc. Natl. Acad. Sci., USA, 80, 1845 (1983)] and Smith et al. [J.W. Smith et al., J. Biol. Chem. 262, 11964, (1987)], a gene (TGC) coding for cysteine was converted to a gene (AGC) coding for serine. In a similar manner as described above, four DNAs: 
A296 5′ GATCGTTGCCAAACTGAACAGCCGACTCTATCGAAA
AGCCAACAAAT 3′,
 A297 3' CAACGGTTTGACTTGTCGGCTGAGATAGCTTTT 5', A298 5′ CCTCCAAGTTAGTGTAATAAG 3′, 
A299 3′ CGGTTGTTTAGGAGGTTCAATCACATTATTCAG
CT 5′
 were synthesized. According to the above-mentioned Ikehara et al. method, these synthetic DNAs were phosphorylated, annealed, and ligated with a DNA fragment excised from pBRSOD1, using BamHI and SalI, to construct pBRHBSOD3. This plasmid had a structure such that a gene for an SOD and a heparin-binding peptide joined to each other was excised by EcoRI and SalI (Fig. 1).

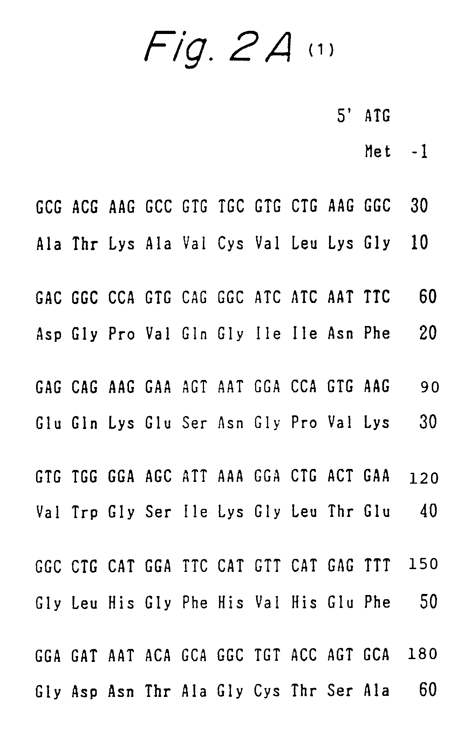

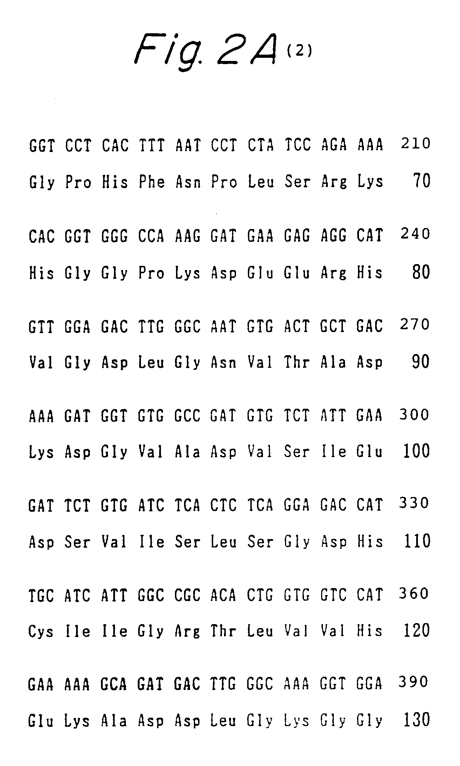

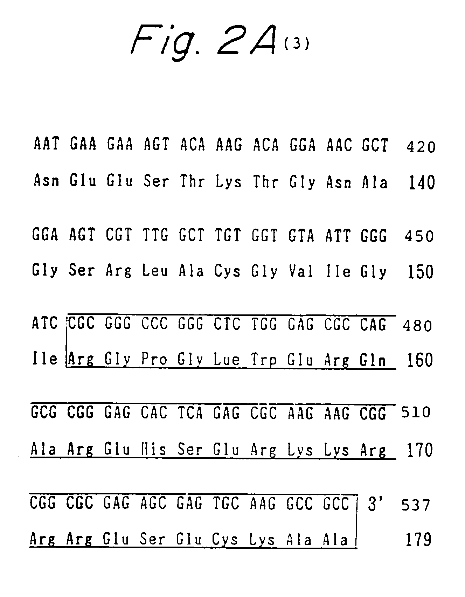

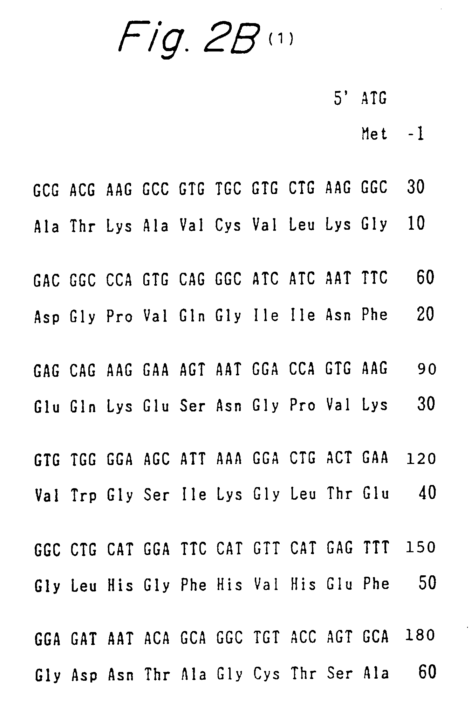

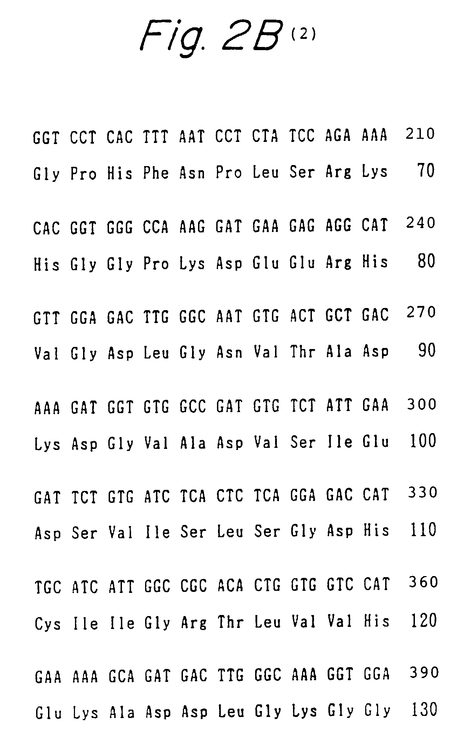

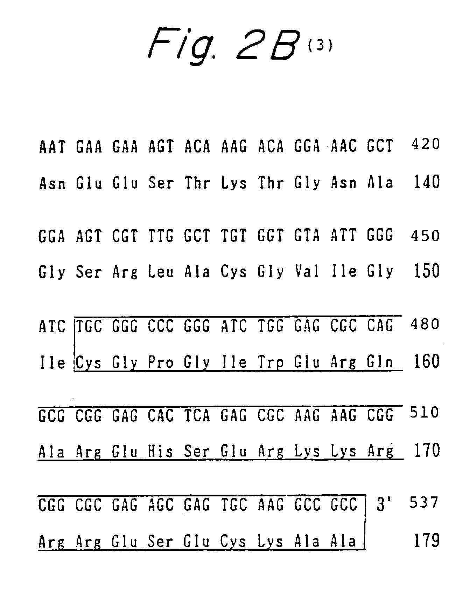

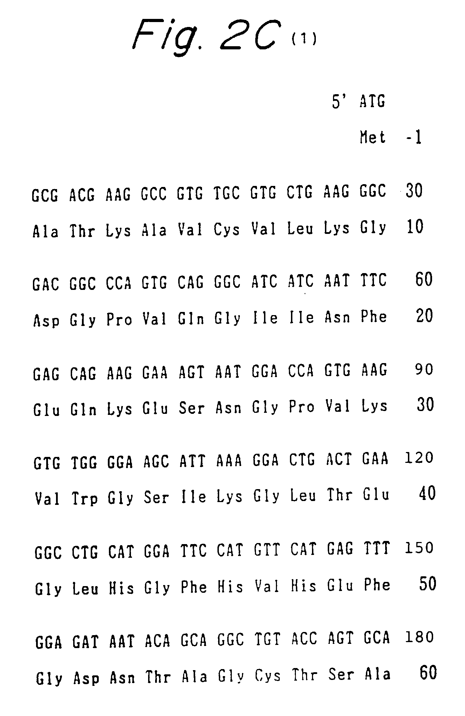

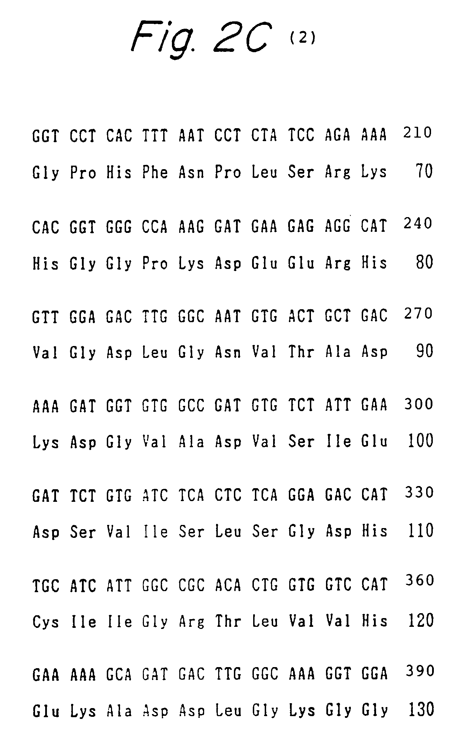

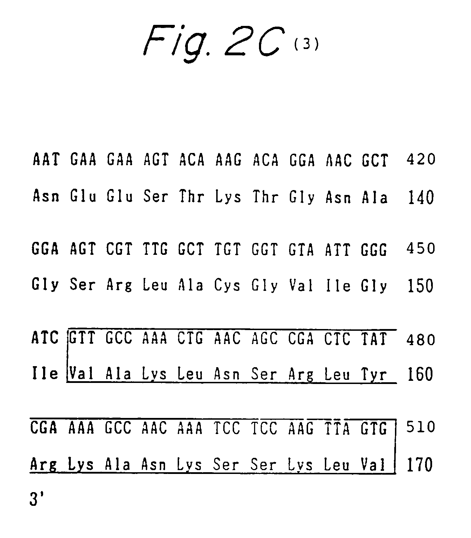

The nucleotide sequences of coding regions and amino acid sequences coded thereby for HB-SOD in pBRHBSOD1, pMHBSOD2 and pBRHBSOD3 are shown in Fig. 2, A to C. In these sequences, boxed portions show heparin-binding domains.

As described above, three genes for HB-SOD having a heparin-binding domain were constructed.

### Example 2. Expression of HB-SOD in yeast

An HB-SOD gene constructed in Example 1 was inserted in a yeast expression plasmid. As a yeast expression plasmid, pYHCC101 (see Japanese Unexamined Patent Publication (KOKAI) No. 01-037291 was used. Note, yeast transformed with the pYHCC101 was designated Saccharomyces cerevisiae SAM0750 and deposited with the Fermentation Research Institute Agency of Industrial Science and Technology on July 16, 1987, as FERM P-9475, and transferred to an international deposition under the Budapest Treaty on February 23, 1990, as FERM BP-2767).

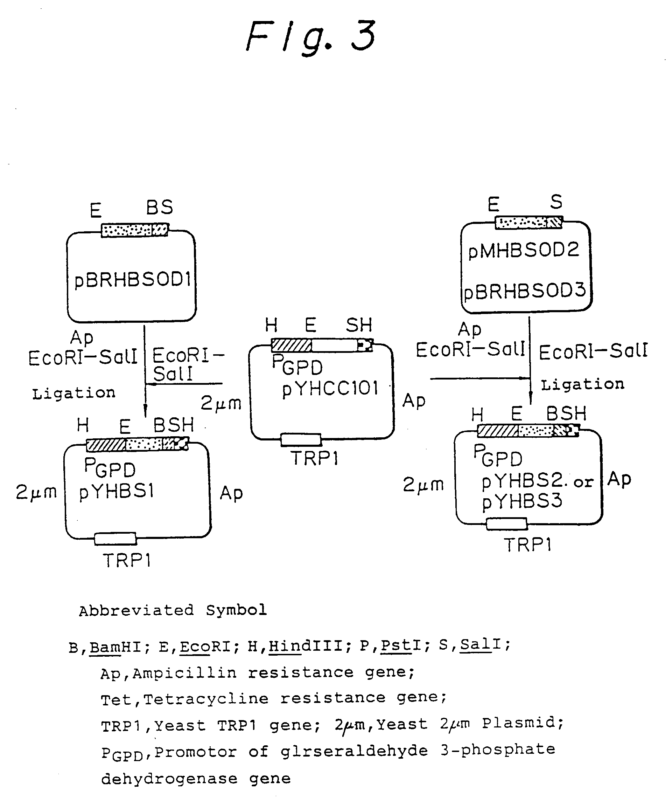

A DNA fragment of about 500 bp obtained from a digestion of pBRHBSOD1 with EcoRI and SalI was ligated with a DNA fragment of about 8 kb obtained from a digestion of pYHCC101 with EcoRI and SalI, to obtain a plasmid designated pYHBS1. This plasmid was amplified using E. coli DH1, and used to transform a yeast strain G-1315 (Mat α, trpl) [H. Yoshizumi et al., J. Jpn. Soc. Starch Sci. 34, 148 (1987)]. The transformation method was that of Ito et al. [Ito et al., J. Bacteriol. 153, 163 (1983)], and a transformant having a restored tryptophan synthesis ability was obtained. Hereinafter this transformant is designated G-1315 (pYHBS1) (Fig. 3).

Moreover, in a similar manner as described above, a DNA fragment containing an HB-SOD gene excised from pMHBSOD2 or pBRHBSOD3 using EcoRI and SalI was inserted into pYHCC101 to obtain pYHBS2 and pYHBS3 respectively, which were then introduced in a yeast strain EH1315 to obtain transformants G-1315 (pYHBS2) and G-1315 (pYHBS3) respectively (Fig. 3).

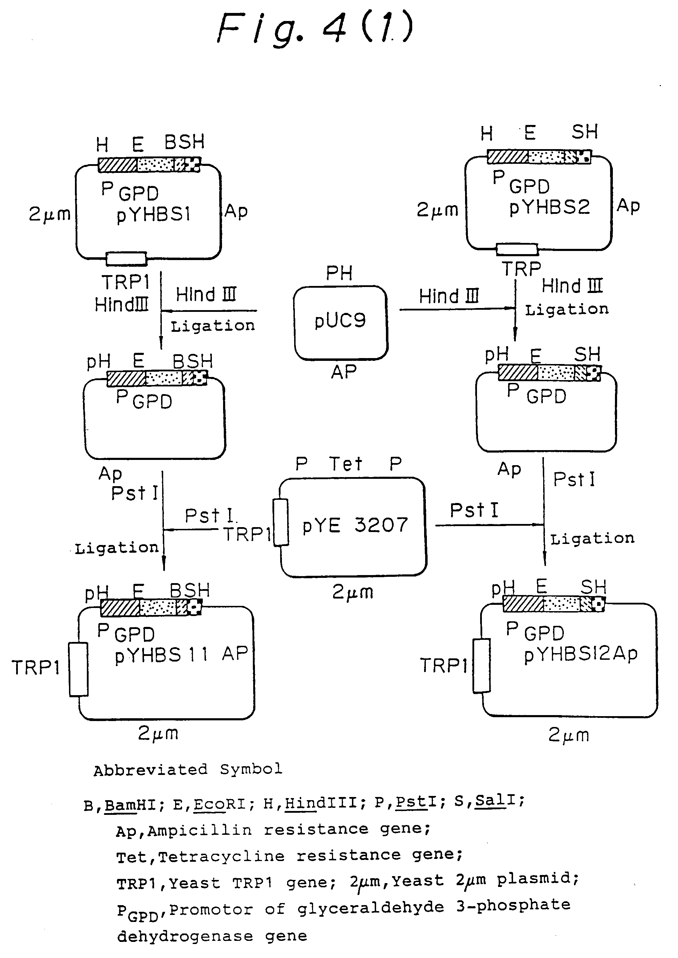

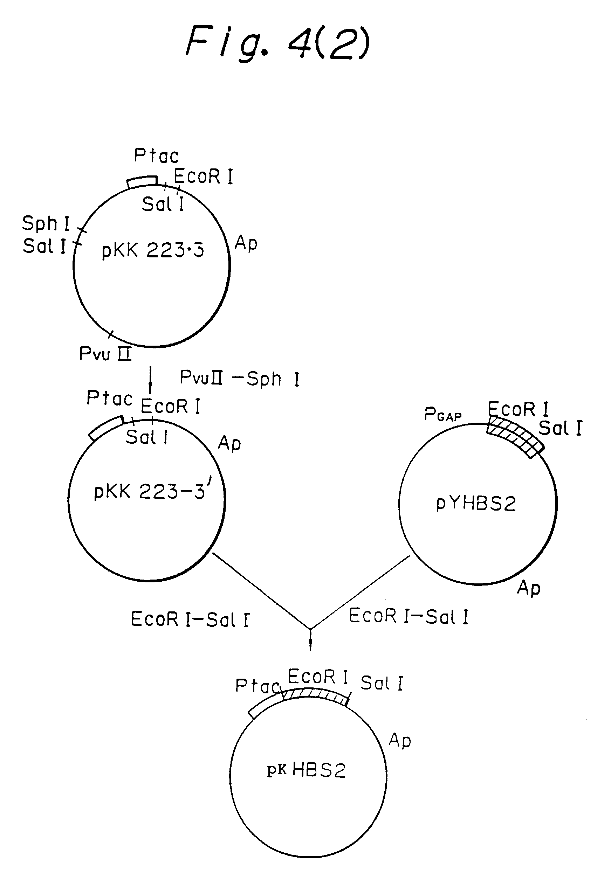

The above-mentioned pYHBS1, pYHBS2 and pYHBS3 are yeast YEp-1 type plasmids. It was confirmed by the following procedure that, in a similar manner as described above, transformants can be obtained using a YEp-2 type plasmid [Takanami et al., Zoku Kagaku Jikken Koza, Idenshikenkyu HoII, Tokyo Kagaku Dojin (1986)] which is said to be more stable than the YEp-1 type (Fig. 4). After digesting pYHBS1 with HindIII, a HindIII fragment containing the HB-SOD gene was recovered, and subcloned into a HindIII site in pUC9. The resulting plasmid was digested with PstI, and subcloned into a PstI site of plasmid pYE3207 [see Japanese Unexamined Patent Publication (KOKAI) No. 61-56077; the pYE3207 was constructed from pJDB219 (Beggs, J.D. Nature 275: 104, 1978; constructed from yeast 2 µm plasmid and pBR322) and YRp7 (ATCC 37060) (Struhl, K. Proc. Natl. Acad. Sci. US. 76: 1035, 1979] to obtain pYHBS11. In a similar manner, after digesting pYHBS2 with HindIII, a HindIII fragment containing an HB-SOD gene was recovered, and subcloned into s HindIII site in pUC9. The resulting plasmid was digested with PstI, and subcloned into a PstI site in plasmid pYE3207 to obtain pYHBS12. Transformants of G-1315 with these plamids were designated G-1315 (pYHBS11) and G-13l5 (pYHBS12) respectively.

The above-mentioned yeast vector is an example of the production of HB-SOD, and as a yeast promoter, a promoter of phosphoglycerokinase gene, or a promoter of repressible acid phosphatase gene, or the like, can be also used.

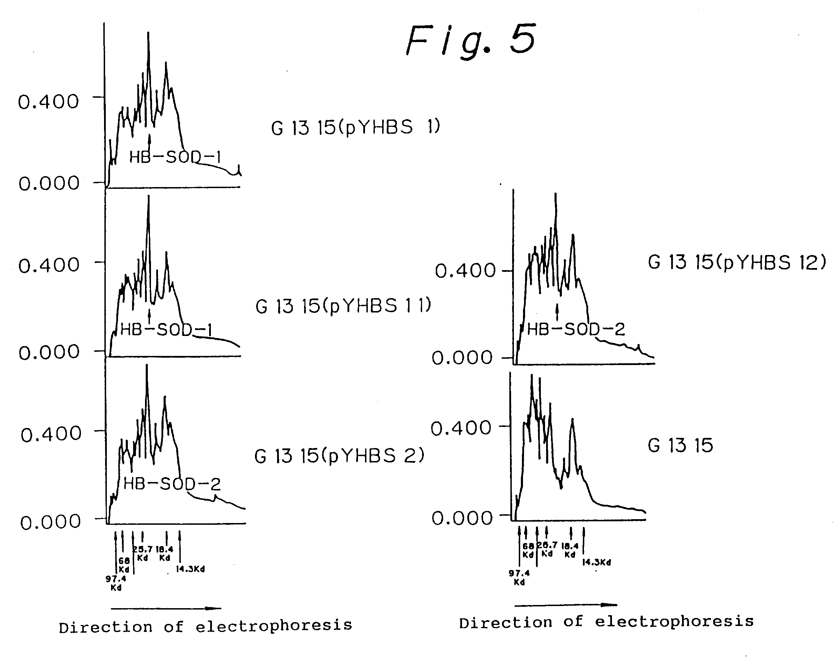

The five transformants thus obtained, i.e., G-1315 (pYHBS1), G-1315 (pYHBS2), G-1315 (pYHBS11), and G-1315 (pYHBS12), were cultured in 2 ml of Burkholder medium [P.R. Burkholder, Am. J. Bot., 30, 206, (1943)], at 30°C for two days with shaking. Cells were collected from 1.5 ml of the broth, and proteins were extracted from the yeast cells according to the Yaffe et al. method [M.P. Yaffe et al. Proc. Natl. Acad. Sci. USA, 81, 4819 (1984)] and was subjected to an SDS polyacrylamide gel electrophoresis. After the electrophoresis, proteins were stained with Coomassie Brilliant Blue R250 [for example, Zoku Seikagaku Jikken Koza Tanpakushitsu No Kagaku, Tokyo Kagaku Dojin (1987)], and as a result, a clear protein band, which was not found for G-1315 not containing plasmid, was observed for all transformants. This band corresponded to about 5% of cellular proteins. The molecular weight thereof was about 21,000, and roughly conformed with an expected molecule for HB-SOD. A result obtained by scanning the gel with a Shimazu chromatoscanner CS-930 is shown in Fig. 5. Moreover, a protein band corresponding to a molecular weight of about 18,000, which was not found for G-13l5 was observed for a similarly cultured G-1315 (pYHBS3). Moreover, in Western blotting using an anti-human erythrocyte SOD antibody, a detection of a product reactive with the antibody was carried out by an enzyme-immuno-assay [for example, Zoku Seikagaku Jikken Koza Tanpakushitsu No Kagaku, Tokyo Kagaku Dojin (1987)]. As a result, since a single band corresponding to HB-SOD developed by the protein staining was observed, it was concluded that HB-SOD was produced in a recombinant yeast. The HB-SOD produced by G-1315 (pYHBS1) and G-1315 (pYHBS11) was designated, HB-SOD-1; the HB-SOD produced by G-1315 (pYHBS2) and G-1315 (pYHBS12) was designated HB-SOD-2; and the HB-SOD produced by G-1315 (pYHBS3) was designated HB-SOD-3.

### Example 3. Purification of HB-SOD

### [Purification Method A]

A piece of cell mass of G-1315 (pYHBS1) on a slant culture was inoculated in 5 ml of Burkholder medium, and cultured at 30°C for 40 hours with shaking. The culture was inoculated to 400 ml of Burkholder medium in a 1 I Erlenmeyer flask, and culturing was similarly carried out. Next, the culture was inoculated in 10 ℓ of Burkholder medium containing 1 mM cupric sulfate and zinc sulfate in a jar fermenter, for 20 hours, and cells were collected by centrifugation to obtain 300 g of cells.

The cells were suspended in 300 ml of 20 mM Tris-HCl buffer (pH 7.5) containing 1 mM cupric sulfate, 1 mM zinc sulfate, 10 mM β-mercaptoethanol and 1 mM (para-amidiphenyl) methanesulfonylfluoride hydrochloride (APMSF), and disrupted by a Dyno Mill. The disrupted cells were centrifuged, and the supernatant was thoroughoutly dialyzed against a 10 mM sodium acetate buffer (pH 4.6) containing 10 mM β-mercaptoethanol and 100 µm APMSF. After the dialysis, the solution was centrifuged, and the supernatant was adsorbed on an SP-Sephadex C-50 (Pharmacia) column (2.5 x 20 cm) equilibrated with the same buffer. The column was washed with 500 ml of a 5 mM potassium phosphate buffer (pH 6.5) containing 10 mM β-mercaptoethanol and 100 µM APMSF, and then elution was carried out using a 10 mM Tris-HCl (pH 8.0) buffer containing 10 mM β-mercaptoethanol, 100 µM APMSF, and 100 mM NaCl. This fraction was heated at 70°C for 10 minutes, centrifuged, and the supernatant was recovered. Then, after an addition of the same volume of water containing 1 mM β-mercaptoethanol and 100 µM APMSF, the supernatant was adsorbed to a column (2.5 x 15 cm) filled with heparin Sepharose CL-6B (Pharmacia) equilibrated with a 10 mM potassium phosphate buffer containing 10 mM β-mercaptoethanol and 100 µM APMSF. After the adsorption, the column was washed with 200 ml of a 10 mM phosphate buffer (pH 7.0) containing 10 mM β-mercaptoethanol, 100 µM APMSF, and 150 mM NaCl. Next, an elution was carried out using a 10 mM phosphate buffer (pH 7.0) containing 1 mM β-mercaptoethanol, 100 µM APMSF, and 500 mM NaCl, and an obtained SOD fraction was applied to a Sephadex G-75 column (2 x 120 cm) equilibrated with 20 mM phosphate buffer (pH 7.0) containing 10 mM β-mercaptoethanol, 100 µM APMSF and 150 mM NaCl, to recover an SOD fraction. As described above, the fraction was subjected to SDS polyacrylamide gel electrophoresis, and proteins were stained. Since a single bond was observed, it was believed that pure HB-SOD-1 was obtained by the above mentioned purification.

According to the same procedure, HB-SOD-2 and HB-SOD-3 were obtained as pure preparations, by a culture of the recombinant yeast and recovery from the cells. Note, purification of HB-SODs was carried out at 4°C.

### [Purification Method B)

Moreover, it was confirmed that a pure preparation can be obtained, according to the following procedure, from an HB-SOD producing strain cultured by the same procedure as described above. Namely, to about 1 kg of cells of HB-SOD-2 producing strain was added 2 ℓ of a 50 mM Tris-HCl buffer (pH 8.8) containing 1 mM cupric sulfate, 1 mM zinc sulfate, 10 mM β-mercaptoethanol, 1 mM APMSF and 1 M NaCl, and the cells were suspended and disrupted with a Dyno-Mill. After eliminating a precipitate by centrifugation, the supernatant was heated at 70°C for 10 minutes. After centrifugation, to the resulting supernatant was added ammonium sulfate to a 50% saturation, and after the ammonium sulfate was completely dissolved, a supernatant was obtained by centrifugation. The supernatant was adsorbed on a butyl Toyopearl (Toyosoda) column (7.5 x 20 cm) equilibrated with 20 mM potassium phosphate buffer (pH 7.0) containing ammonium sulfate at a 50% saturation and 1 mM β-mercaptoethanol. The column was washed with the same buffer and eluted with a 20 mM potassium phosphate buffer (pH 7.0) containing ammonium phosphate at a 30% saturation and 1 mM β-mercaptoethanol. A fraction having an SOD activity was recovered and desalted by a Sephadex G-25 (Pharmacia) column (30 x 90 cm) equilibrated with a 10 mM Tris-HCl buffer (pH 8.5) containing 50 mM NaCl. The desalted fraction was allowed to pass through a DEAE-Sepharose (Pharmacia) column (5 x 10 cm) equilibrated with the same buffer. The active fraction was adjusted to pH of 7.0 by HCl, and allowed to pass through an SP-Sephadex A-50 column (5 x 10 cm). The SOD fraction was then adsorbed to a heparin Sepharose CL-6B column (10 x 10 cm). The column was washed with 1 ℓ of 10 mM sodium phosphate buffer (pH 7.0) containing 150 mM NaCl, and eluted with 10 mM sodium phosphate buffer (pH 7.0) containing 500 mM NaCl.

### Example 4. Expression in E. coli

An expression of HB-SOD in E. coli was studied. Namely, an E. coli expression vector pKK223-3 (Pharmacia) was digested with PvuII and SphI, and the larger fragment among two resulting fragments was recovered, blunt-ended using T4 polymerase, and ligated. A resulting plasmid was designated pKK223-3'. The plasmid pKK223-3' was digested with EcoRI and SalI, the larger DNA fragment among the resulting fragments was recovered and ligated with a 450 bp DNA fragment obtained by digesting pYHBS2 with EcoRI and SalI, to obtain a plasmid pKHBS2 which was then used to transform E. coli JM109. A transformant, which had acquired an ampicillin resistance, was cultured in an L-medium (1% polypepton, 0.5% yeast extract, 0.5% NaCl, pH 7.0) containing 1 mM IPTG, and a protein fraction was obtained according to the above-mentioned procedure and applied to SDS polyacrylamide gel electrophoresis, and as a result, a band for HB-SOD was observed. The corresponding band was not observed for a strain not containing the plasmid. Moreover, it was confirmed that this band was reactive with anti-human SOD antibody, by an enzyme-antibody assay, and therefore, it was demonstrated that HB-SOD was also expressed in E. coli.

### Example 5. Properties of HB-SOD

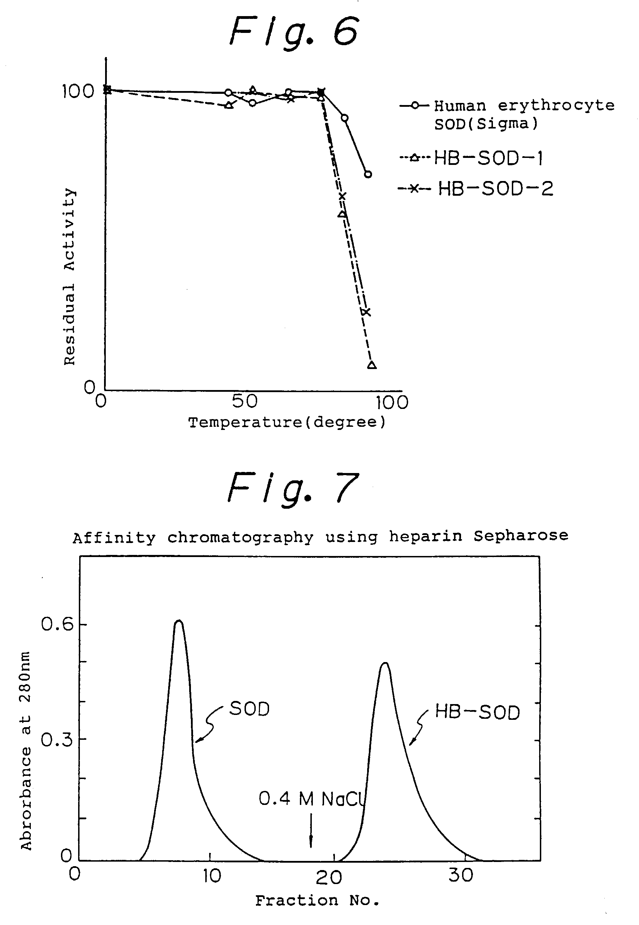

First, the thermostability of a recombinant HB-SOD was tested. Namely, 100 µl of a solution of about 50U/ml HB-SOD-1, HB-SOD-2 and human erythrocyte SOD (Sigma) in a 10 mM phosphate buffer (pH 7.0) was put into an Eppendorf tube, incubated in a water bath at each temperature for 10 minutes, cooled in ice, and the remaining activity measured. The result is shown in Fig. 6. It is known that SOD is a protein which is very thermostable, and in this experiment, the activity was not lowered by heating at 72°C for 10 minutes. On the other hand, HB-SOD-1 and HB-SOD-2 are also resistant against heating at 72°C for 10 minutes. Upon heating at 80°C far 10 minutes, although human SOD is more stable, HB-SOD-1 and HB-SOD-2 also maintain a substantial activity, and both the HB-SODs were thermostable enzymes.

An amino acid analysis of HB-SOD-2 and HB-SOD-3 purified according to the procedure described in Example 4 was carried out, and the results are shown in Tables 1 and 2. The analytical values closely conform to those expected from the nucleotide sequence of DNA.

**Table 1**

| Amino acid analysis | | |
|---|---|---|
| HB-SOD-2 | | |
| Amino acid | Value expected from nucleotide sequence | Analytical value |
| Asp | 11 | 18.5 |
| Asn | 7 | |
| Thr | 8 | 7.9 |
| Ser | 12 | 11.4 |
| Glu | 15 | 17.8 |
| Gln | 3 | |
| Pro | 6 | 5.8 |
| Gly | 27 | 26.5 |
| Ala | 12 | 11.3 |
| Cys | 6 | Not determined |
| Val | 14 | 14.0 |
| Met | 0 | 0 |
| Ile | 10 | 9.8 |
| Leu | 9 | 8.7 |
| Tyr | 0 | 0 |
| Phe | 4 | 3.8 |
| His | 9 | 9.2 |
| Lys | 14 | 14.2 |
| Arg | 10 | 9.9 |
| Trp | 2 | Not determined |

**Table 2**

| Amino acid analysis | | |
|---|---|---|
| HB-SOD-3 | | |
| Amino acid | Value expected from nucleotide sequence | Analytical value |
| Asp | 11 | 18.0 |
| Asn | 9 | |
| Thr | 9 | 8.3 |
| Ser | 13 | 12.9 |
| Glu | 10 | 12.8 |
| Gln | 2 | |
| Pro | 5 | 4.9 |
| Gly | 25 | 25.2 |
| Ala | 11 | 11.1 |
| Cys | 4 | Not determined |
| Val | 16 | 14.9 |
| Met | 0 | 0 |
| Ile | 9 | 7.5 |
| Leu | 12 | 10.8 |
| Tyr | 1 | 0.8 |
| Phe | 4 | 4.1 |
| His | 8 | 8.2 |
| Lys | 15 | 14.1 |
| Arg | 6 | 6.0 |
| Trp | 1 | Not determined |

Moreover, although an amino-terminal amino acid sequence was determined by a gas phase sequencer (Applied Biosystems 470A), a PTH amino acid was not detected; suggesting that the amino terminal was blocked. The amino terminal of native human erythrocyte SOD was acetylated, and a recombinant human erythrocyte SOD produced in yeast was also acetylated (R.A. Hallewell et al., Bio/Technology, 5, 366 (1987)). From the above, the HB-SOD-2 has an expected primary structure.

Purified HB-SOD was subjected to gel filtration column chromatography using Sephadex G-75 (Pharmacia), and as a result, the apparent molecular weight was found to be about 40,000, and thus it was considered that it formed a dimer in the same way as human erythrocyte SOD.

The specific activity of HB-SOD-2 was measured by the above-mentioned chytocrome C method, and an amount of protein was measured by a BCA protein quantitation kit. As a control, a specific activity of human erythrocyte SOD (Sigma) was measured. Purified HB-SOD-2 was calcinated by a Hitachi GA/3 graphite atomizer, and copper and zinc contained in HB-SOD-2 were quantitated by a Hitachi 180/50 absorptiometer. These results are summarized in Table 3. It was found that the HB-SOD, having a structure which contains an artificially added heparin-binding peptide, includes coordinated copper and zinc, and exhibits an SOD activity.

**Table 3**

| Specific activity of HB-SOD and metals contained therein | | | |
|---|---|---|---|
| | Specific activity (U/mg) | Copper | Zinc |
| HB-SOD-2 | | | |
| (Lot 1) | 778 | 0.435 | 1.85 |
| (Lot 2) | 945 | 0.392 | 1.20 |
| (Lot 3) | 567 | 0.299 | 0.90 |
| Human erythrocyte SOD | 2700 | 0.807 | 1.32 |

The human erythrocyte SOD was obtained from Sigma.

A specific activity of HB-SOD-2 obtained by the purification method B was determined by the same procedure, and was found to be 2400 U/mg. A specific activity of the human erythrocyte SOD was 2700 U/mg.

### Example 6. Heparin binding property of HB-SOD

HB-SOD-2 radioactively labeled with ¹²⁵I (20,000 cpm) (see, J. Pongou, Method in Enzymology, 70, 22: 1987), dissolved in 10 mM phosphate buffer containing 150 mM NaCl, was adsorbed to 2 ml of heparin-Sepharose CL-6B. Under this condition, HB-SOD was properly adsorbed to the heparin-Sepharose CL-6B, and was eluted by increasing the NaCl concentration to 0.4 M. On the other hand, although human SOD was subjected to the adsorption on the other column, under the same condition, it was not adsorbed, but passed through. This result is shown in Fig. 7. A volume of each fraction was 1 ml, and the chromatography was carried out at a room temperature.

### Example 7. Metabolism of HB-SOD in blood

The concentration in the blood of the radioactively labeled HB-SOD-2 and human erythrocyte SOD was measured, after an intravenous administration thereof.

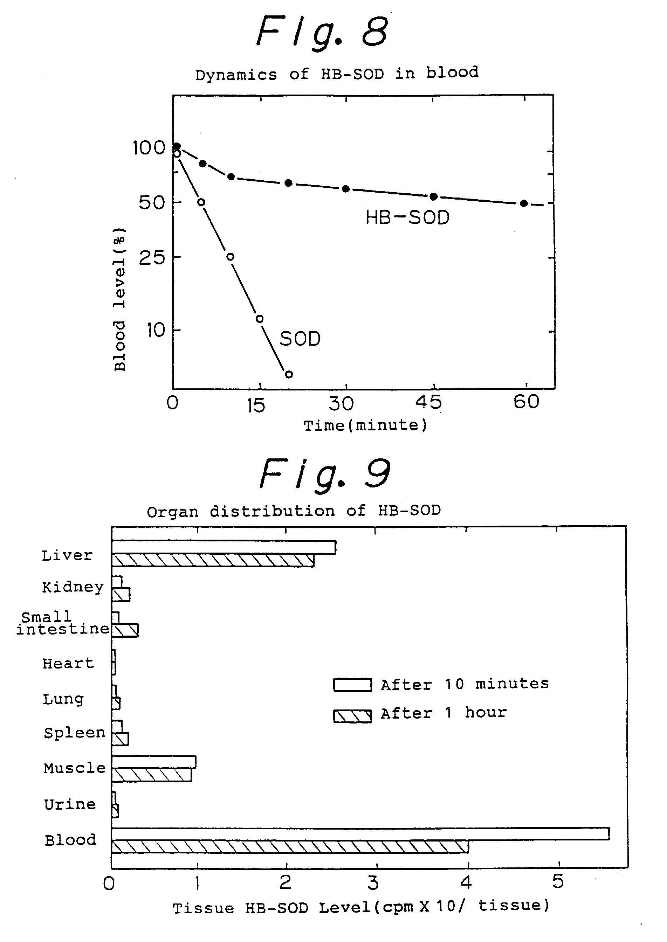

In this measurement, 20 µg of the radioactively labeled HB-SOD-2 or human erythrocyte SOD was dissolved in a physiological saline, the solution was injected to a Wister rat (body weight 200 g) through a tail vein, and a concentration in the blood of the HB-SOD-2 or SOD was measured over a lapse of time [see, Inoue et al., Rinsho of Free Radical (Nippon Igaku Kan) 2, 83 (1987)]. The result is shown in Fig. 8.

Human SOD has a half time of a few minutes, and rapidly disappears from the blood. On the other hand, it was found that HB-SOD is stably present in the blood for a long time. This means that an activity eliminating active oxygen is stably present in the blood for a long time, and demonstrates the usefulness of the present HB-SOD.

### Example 8. Distribution of HB-SOD between organs

To test the distribution of HB-SOD between organs, radioactively labeled HB-SOD was injected to a rat in the same manner as described above, and 10 minutes or one hour later the rat was killed and the radioactivity of each organ was measured. The result is shown in Fig. 9. It was found that the HB-SOD was distributed in the liver and muscles, in addition to the blood. This is an advantageous property of the present HB-SOD, and this property is not found in human erythrocyte SOD. Moreover, although HB-SOD in the blood decreased by about 20% with a lapse of time. There was little decrease in peripheral organs, and the HB-SOD increased in the small intestine and spleen. It is considered that HB-SOD is stably present in each organ by binding to endotherial cells of blood vessels of the organ, and this is also an advantageous property not found in human erythrocyte SOD. It is considered that an amount of HB-SOD administered to obtain a same effect is very low, in comparison to that of human erythrocyte SOD.

The superoxide-eliminating activity in endotherial cells of a blood vessel can be maintained for a long time. Especially, since an accumulation of HB-SOD in the liver, which contains a large amount of heparin sulfate, is remarkable, it can be used for an elimination of free radicals in the liver and is useful against, for example, ischemic liver disease.

### Example 9. Inhibition effect of HB-SOD against Ischemia reperfusion arrthymia

It is considered that ischemia reperfusion arrthymia occurs by reperfusion of the blood containing oxygen to a site of an abnormal metabolism topically induced by an oxygen deficiency. Therefore, it was determined whether HB-SOD-2 is effective against this arrythmia [see, Protein, Nucleic Acid and Enzyme, 33, 2889 (1988)].

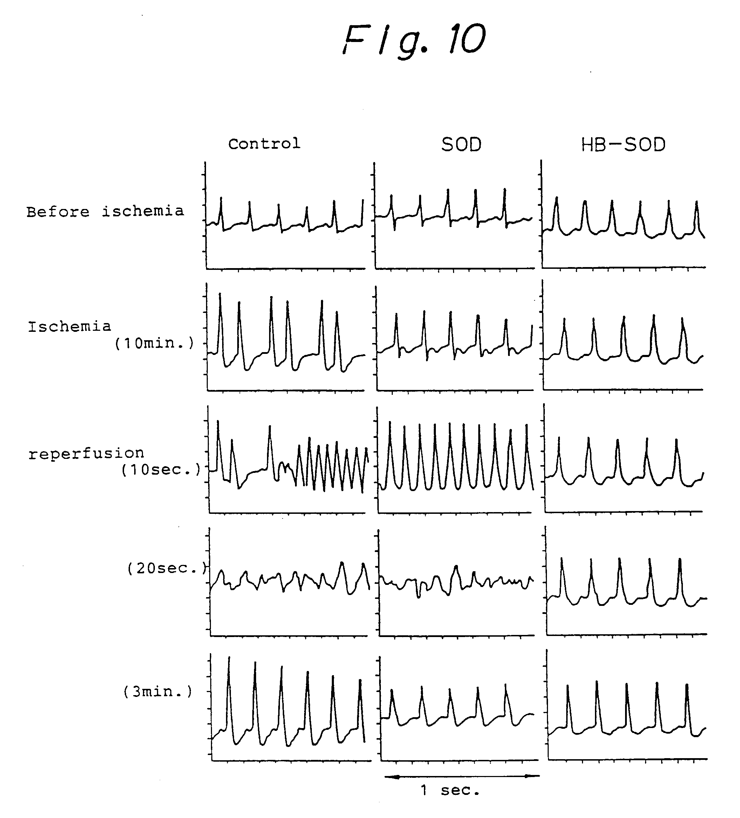

First, in a rat anesthesized with pentobarbital, the left coronary artery descending branch was closed for 10 minutes, and then was re-opened. In this case, where the cardiac function was continuously monitored by an electrocardiogram, a remarkable arrthymia occurred after a reperfusion of the blood (Fig. 10, on the left). When HB-SOD-2 had been intraveneously administrated (5 mg/kg), this arrhythmia was inhibited (Fig. 10, on the right). Under the same condition, human erythrocyte SOD did not exhibit such a protective effect (Fig. 10, at the center). Moreover, when HB-SOD-3 had been intravenously administrated (5 mg/kg), the arrhythmia was effectively inhibited (Table 4). It is considered that HB-SOD prevents arrhythmia by eliminating superoxide radicals generated by a ischemia reperfusion.

**Table 4**

| Treatment (n the number) | PVC (times/3 min) | Frequency (%) | | Duration time (sec.) | |
|---|---|---|---|---|---|
| | | VT | Vf | VT | Vf |
| Not treated (22) | 27 ± 7 | 100 | 31.8 | 23.8 ± 2.2 | 1.3 ± 3.5 |
| SOD administration (15) | 21 ± 3 | 100 | 13.3 | 19.1 ± 2.2 | 1.3 ± 1.1 |
| HB-SOD administration (12) | 7 ± 2 | 58.3 | 8.3 | 5.9 ± 2.3 | 0.3 ± 0.1 |

- PVC: Premature ventricular contraction
- VT: Ventricular tachycardia
- Vf: Ventricular fibrilation

### Example 10. Inhibition effect of HB-SOD against fatal arrhythmia

In a rat anesthesized with pentobarbital, the left cornary artery descending branch was permanently ligated. Although in the non-treated group, about 60% of rats died from ventricular fibriliation, where 10 mg/kg of HB-SOD-3 had been once intravenously administered 15 minutes before the coronary artery ligature, the arrhythmia was effectively inhibited (Table 5).

**Table 5**

| Treatment (n the number) | PVC (times/3 min) | Duration time | | Mortality(%) |
|---|---|---|---|---|
| | | VT | Vf | |
| Control (12) | 106 ± 23 | 124.3 ± 30.7 | 623.1 ± 155.5 | 58.3 |
| HB-SOD administration (11) | 230 ± 90 | 101.4 ± 21.7 | 228.8 ± 141.2 | 18.1 |

- PVC: Premature ventricular contraction
- VT: Ventricular tachycardia
- Vf: Ventricular fibrilation

### Example 11. Inhibition effect of HB-SOD against stress stomach ulcer [See hirota et al., Morbid State of Stamach Mucosa and Free Radical (Nippon Iqakukan) 63, (1987)]

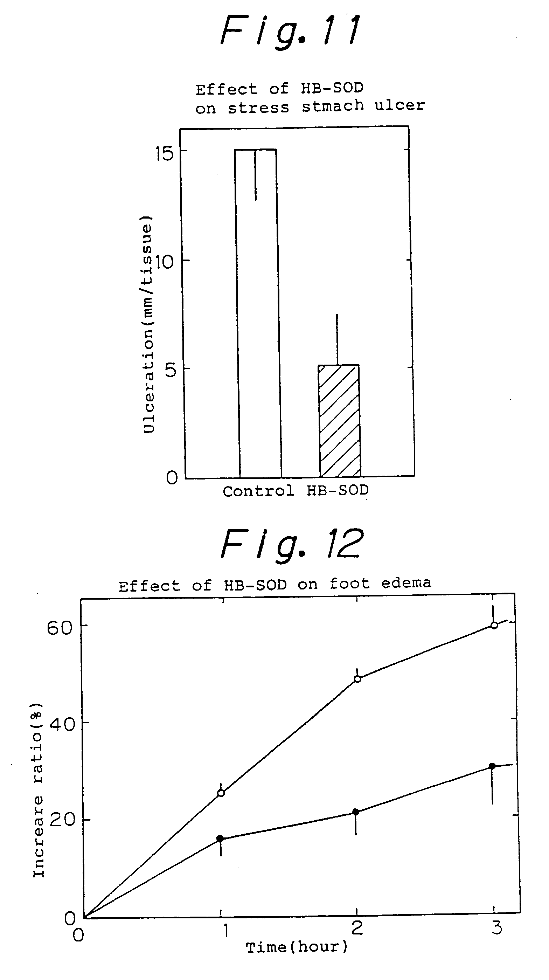

When a rat has received vestraint and water immersion stress (22°C, 6 hours) without anesthesia, remarkable stomach mucous membrane lesion developes with a lapse of time (Fig. 11, on the left). This mucous membrane lesion was remarkably inhibited by previously intravenously administering HB-SOD-2 (5 mg/kg) (Fig. 11, on the right). Under the same condition, a native SOD did not exhibit this protective effect.

Therefore, the present HB-SOD is also effective against stomach ulcers.

### Example 12. Inhibition effect of HB-SOD against carragenin-induced foot edema

In a rat under ether anesthesia, when 0.1 ml of suspension of carragenin in physiological saline was subcutaneously administrated into a foot (10 mg/kg), the volume of the foot increased with a lapse of time (Fig. 12, o). On the other hand, when HB-SOD-2 had been previously intraveneously administered (5 mg/kg), the lesion was remarkably inhibited or regressed (Fig. 12, •). Under the same condition, native SOD did not exhibit this protective effect.

The above-mentioned pharmacological effects of the HB-SOD are summarized in Table 6, in comparison with those of human erythrocyte SOD.

**Table 6**

| Tissue protective effects of HB-SOD | | |
|---|---|---|
| Lesion model | Lesion development inhibition effect | |
| | SOD | HB-SOD |
| Traumatic brain edema | - | + |
| Ischemic myocardial disease | - | + |
| Stomach ulcer | - | + |
| Edema | - | + |
| Ishemic liver disease | - | + |

### Example 13. Protective effect of HB-SOD against brain edema

The protective effect of HB-SOD-2 against brain edema was tested according to the Ando et al. method (Y. Ando et al., Brain Res. 477, 286 (1989)3. Namely, 0.2 ml of 1% Evans' Blue was intraveneously administered to a rat under pentobarbital anesthesis, and 20 g of clay treated with liquid nitrogen was placed in contact with the right hemicerebrum from outside of the cranium, to induce traumatic brain edema. After 30 minutes from the start of the treatment, the hemicerebrum was removed, a dye was extracted with 5 ml of dimethylformamide for 24 hours, and was measured by colorimetry. The results are shown in Table 7.

**Table 7**

| Treatment | Dose | Evans' Blue (µmol/30 min.) |
|---|---|---|
| Non treatment | - | 1.25 |
| Human erythrocyte SOD | 5 mg/kg | 1.20 |
| HB-SOD-2 | 5 mg/kg | 0.50 |

* In the Table, "Human erythrocyte SOD" and "HB-SOD-2" mean that corresponding SOD had been previously intraveneously administered.

Although 1.25 µmol/30 min. Evans' Blue was detected in the non-treatment group, only 0.50 µmol/30 min was detected in the HB-SOD-2 group, revealing that HB-SOD-2 exhibits remarkable protective effects against edema.

### Example 14. Protective effect of HB-SOD against hepatic insufficiency

In a rat under pentobarbital anesthesis, the portal vein was ligated for 20 minutes, and after one hour, the blood was again allowed to flow to induce a ischemia reperfusion disease in the liver. Then 5 µmol/kg of sulfobromophthalein (BSP) was intraveneously administered to this rat, and an amount of BSP excreted into the bile for 30 minutes was spectrometrically measured according to the Inoue et al. method [M. Inoue et al., Proc. Natl. Acad. Sci. USA, 80, 7654 (1983)]. The results are shown in Table 8.

**Table 8**

| Test group | Dose | Percent excretion of BSP into bile |
|---|---|---|
| Control | - | 45% |
| Human erythrocyte SOD | 5 mg/kg | 48% |
| HB-SOD-2 | 5 mg/kg | 88% |

* In the Table, "human erythrocyte SOD" and "HB-SOD-2" mean that the corresponding SOD had been previously intraveneously administrated.

Although the human erythrocyte SOD was ineffective, HB-SOD provided a remarkable protective effect, as demonstrated by the 88% excretion ratio, revealing that HB-SOD is also effective against hepatic insufficiency.

### INDUSTRIAL APPLICABILITY

An HB-SOD gene constructed by joining a gene coding for a heparin-binding peptide to an SOD gene, according to the present invention, was expressed in yeast to obtain HB-SOD having a novel structure. The HB-SOD has an SOD activity and heparin-binding ability. By using such procedure, not only heparin-binding peptides but also other SODs having a novel structure and function can be artificially designed by binding various biologically active peptides using a gene technology or protein technology.

The HB-SODs obtained according to the present invention have a very long half-time in the blood in comparison to native SOD, and have a tendency to a tropism to the liver, etc. In these points, the HB-SODs are advantageous over native SOD. Moreover, the HB-SODs are remarkably effective against traumatic brain edema, and ischemic myocardial disease, stomach ulcer, edema, and ischemic liver disease, against which native SOD is not effective.

Although attempts have been made to overcome the drawbacks of native SOD by chemically modifying the native SOD, and chemically modified SOD is foreign to an organism and there is a possibility that the chemically modified SOD per se, or degraded products or metaborites thereof, will provide side effects, since the HB-SOD is a hybrid of SOD and a heparin-binding peptide both having a native structure, the possibility of the HB-SOD per se or a degraded product, thereof, providing side effects is much lower than for a chemically modified SOD. Moreover, the modification of native SOD necessitates a step of a modification of a purified SOD, but according to the present invention, an effective HB-SOD can be obtained without this step. This is another advantageous point of the present invention.

Reference to deposited microorganism under Rule 13-2 and depository institute:
Fermentation Research Institute Agency of Industrial Science and Technology Ministry of International Trade and Industry;
Address: 1-3 Higashi 1-chome, Tsukuba-shi, Ibaraki-ken, Japan;
Deposition number and deposition date:
   1. FERM BP-2767 July 16, 1987

## Claims

1. A method of making a superoxide dismutase having enhanced lifetime in the blood, comprising using DNA recombination to add a heparin-binding domain to a superoxide dismutase originally lacking a heparin-binding domain.

2. A superoxide dismutase comprising CuZn-superoxide dismutase, originally lacking a heparin-binding domain, recombinantly joined to a heparin-binding domain, in which the heparin-binding domain is either of another superoxide dismutase which does have a heparin-binding domain or from human antithrombin III.

3. A superoxide dismutase comprising a superoxide dismutase coded for by human DNA hybridisable with a probe of the sequence: 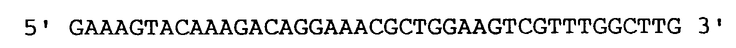 and originally lacking a heparin-binding domain, recombinantly joined to a heparin-binding domain, in which the heparin-binding domain is either of another superoxide dismutase which does have a heparin-binding domain or from human antithrombin III.

4. A superoxide dismutase comprising an intracellular superoxide dismutase with a native sequence lacking a heparin-binding domain, recombinantly joined to a heparin-binding domain, in which the heparin-binding domain is either of another superoxide dismutase which does have a heparin-binding domain or from human antithrombin III.

5. A superoxide dismutase according to any of claims 2, 3 or 4 in which the heparin-binding domain of another superoxide dismutase which does have a heparin-binding domain is of extracellular superoxide dismutase.

6. A superoxide dismutase according to claim 5 in which the heparin-binding domain is a peptide having the amino acid sequence: 
Arg Gly Pro Gly Leu Trp Glu Arg Gln Ala Arg Glu His
Ser Glu Arg Lys Lys Arg Arg Arg Glu Ser Glu Cys Lys
Ala Ala
 or a peptide having substantially the same amino acid sequence and having substantially the same heparin-binding activity.

7. A superoxide dismutase according to claim 5 in which the heparin-binding domain is a peptide having the amino acid sequence: 
Cys Gly Pro Gly Ile Trp Glu Arg Gln Ala Arg Glu His
Ser Glu Arg Lys Lys Arg Arg Arg Glu Ser Glu Cys Lys
Ala Ala
 or a peptide having substantially the same amino acid sequence and having substantially the same heparin-binding activity.

8. A superoxide dismutase according to any of claims 2, 3 or 4 in which the heparin-binding domain from human antithrombin III is a peptide having the amino acid sequence: 
Val Ala Lys Leu Asn Ser Arg Leu Tyr Arg Lys Ala Asn
Lys Ser Ser Lys Leu Val
 or a peptide having substantially the same amino acid sequence and having substantially the same heparin-binding activity.

9. A superoxide dismutase according to any one of claims 2 to 8 in which the superoxide dismutase lacking heparin-binding domain is human erythrocyte superoxide dismutase.

10. Recombinant DNA coding for a superoxide dismutase according to any one of claims 2 to 9.

11. A host transformed with recombinant DNA according to claim 10 in expressible form.

12. A process for the production of recombinant superoxide dismutase, comprising culturing a host according to claim 11 to produce the recombinant superoxide dismutase, and recovering the recombinant superoxide dismutase from the culture.

13. A pharmaceutical preparation comprising a superoxide dismutase according to any one of claims 2 to 9.

14. A pharmaceutical preparation according to claim 13 for the treatment of traumatic brain edema, ischemic myocardial disorder, stomach ulcer, edema or ischemic liver disorder.

15. The use of a superoxide dismutase according to any one of claims 2 to 9 in the preparation of a medicament for the treatment of any one of traumatic brain edema, ischemic myocardial disorder, stomach ulcer, edema or ischemic liver disorder.

## Patentansprüche

1. Verfahren zur Herstellung von Superoxid-Dismutase mit besserer Haltbarkeit im Blut, welches die Anwendung der DNA-Rekombination umfaßt, damit der Superoxid-Dismutase, der ursprünglich eine Heparin bindende Region fehlt, eine Heparin bindende Region hinzugefügt wird.

2. Superoxid-Dismutase, die Cuzn-Superoxid-Dismutase umfaßt, der ursprünglich die Heparin bindende Region fehlt, die rekombinant mit einer Heparin bindenden Region verbunden ist, wobei die Heparin bindende Region entweder von einer anderen Superoxid-Dismutase, die eine Heparin bindende Region hat, oder von Human-Antithrombin III ist.

3. Superoxid-Dismutase, die Superoxid-Dismutase umfaßt, die durch Human-DNA codiert wird, die mit einer Sonde der Sequenz hybridisierbar ist: 5' GAAAGTACAAAGACAGGAAACGCTGGAAGTCGTTTGGCTTG 3', und der ursprünglich die Heparin bindende Region fehlt, die rekombinant mit einer Heparin bindenden Region verbunden ist, wobei die Heparin bindende Region entweder von einer anderen Superoxid-Dismutase, die die Heparin bindende Region aufweist, oder von Human-Antithrombin III ist.

4. Superoxid-Dismutase, die intrazelluläre Superoxid-Dismutase mit einer natürlichen Sequenz umfaßt, der die Heparin bindende Region fehlt, die rekombinant mit einer Heparin bindenden Region verbunden ist, wobei die Heparin bindende Region entweder von einer anderen Superoxid-Dismutase, die die Heparin bindende Region aufweist, oder von Human-Antithrombin III ist.

5. Superoxid-Dismutase nach einem der Ansprüche 2, 3 oder 4, wobei die Heparin bindende Region der anderen Superoxid-Dismutase, die die Heparin bindende Region aufweist, von extrazellulärer Superoxid-Dismutase ist.

6. Superoxid-Dismutase nach Anspruch 5, wobei die Heparin bindende Region ein Peptid mit der Aminosäuresequenz: 
Arg Gly Pro Gly Leu Trp Glu Arg Gln Ala Arg Glu His
Ser Glu Arg Lys Lys Arg Arg Arg Glu Ser Glu Cys Lys
Ala Ala
 oder ein Peptid mit im wesentlichen der gleichen Aminosäuresequenz und mit im wesentlichen der gleichen Heparinbindungsaktivität ist.

7. Superoxid-Dismutase nach Anspruch 5, wobei die Heparin bindende Region ein Peptid mit der Aminosäuresequenz: 
Cys Gly Pro Gly Ile Trp Glu Arg Gln Ala Arg Glu His
Ser Glu Arg Lys Lys Arg Arg Arg Glu Ser Glu Cys Lys
Ala Ala
 oder ein Peptid mit im wesentlichen der gleichen Aminosäuresequenz und mit im wesentlichen der gleichen Heparinbindungsaktivität ist.

8. Superoxid-Dismutase nach einem der Ansprüche 2, 3 oder 4, wobei die Heparin bindende Region von Human-Antithrombin III ein Peptid mit der Aminosäuresequenz: 
Val Ala Lys Leu Asn Ser Arg Leu Tyr Arq Lys Ala Asn
Lys Ser Ser Lys Leu Val
 oder ein Peptid mit im wesentlichen der gleichen Aminosäuresequenz und mit im wesentlichen der gleichen Heparinbindungsaktivität ist.

9. Superoxid-Dismutase nach einem der Ansprüche 2 bis 8, wobei die Superoxid-Dismutase, der die Heparin bindende Region fehlt, Human-Erythrocyt-Superoxid-Dismutase ist.

10. Rekombinante DNA, die die Superoxid-Dismutase nach einem der Ansprüche 2 bis 9 codiert.

11. Wirt, der mit rekombinanter DNA nach Anspruch 10 in ausprägbarer Form transformiert ist.

12. Verfahren zur Herstellung von rekombinanter Superoxid-Dismutase, das die Züchtung eines Wirts nach Anspruch 11, wodurch die rekombinante Superoxid-Dismutase produziert wird, und die Gewinnung der rekombinanten Superoxid-Dismutase aus der Kultur umfaßt.

13. Pharmazeutisches Präparat, das Superoxid-Dismutase nach einem der Ansprüche 2 bis 9 umfaßt.

14. Pharmazeutisches Präparat nach Anspruch 13 für die Behandlung des traumatischen Gehirnödems, einer ischämischen myokardialen Erkrankung, eines Magengeschwürs, eines Ödems oder einer ischämischen Lebererkrankung.

15. Verwendung der Superoxid-Dismutase nach einem der Ansprüche 2 bis 9 bei der Herstellung eines Medikaments zur Behandlung von traumatischem Gehirnödem, ischämischer myokardialer Erkrankung, Magengeschwür, Ödem oder ischämischer Lebererkrankung.

## Revendications

1. Un procédé de préparation d'une superoxide dismutase présentant une durée de vie améliorée dans le sang, comprenant l'utilisation de la recombinaison d'ADN pour ajouter un domaine de liaison à l'héparine à une superoxide dismutase primitivement dépourvue d'un domaine de liaison à l'héparine.

2. Une superoxide dismutase comprenant une CuZn-superoxide dismutase, primitivement dépourvue d'un domaine de liaison à l'héparine, liée par recombinaison à un domaine de liaison à l'héparine, dans laquelle le domaine de liaison à l'héparine provient soit d'une autre superoxide dismutase qui comporte effectivement un domaine de liaison à l'héparine, soit d'une antithrombine III humaine.

3. Une superoxide dismutase comprenant une superoxide dismutase codée par un ADN humain susceptible de s'hybrider avec une sonde présentant la séquence : 5' GAAAGTACAAAGACAGGAAACGCTGGAAGTCGTTTGGCTTG 3' et primitivement dépourvue d'un domaine de liaison à l'héparine, liée par recombinaison à un domaine de liaison à l'héparine , dans laquelle le domaine de liaison à l'héparine provient soit d'une autre superoxide dismutase qui comporte effectivement un domaine de liaison à l'héparine, soit d'une antithrombine III humaine.

4. Une superoxide dismutase comprenant une superoxide dismutase intracellulaire ayant une séquence native dépourvue d'un domaine de liaison à l'héparine, liée par recombinaison à un domaine de liaison à l'héparine, dans laquelle le domaine de liaison à l'héparine provient soit d'une autre superoxide dismutase qui comporte effectivement un domaine de liaison à l'héparine, soit d'une antithrombine III humaine.

5. Une superoxide dismutase selon l'une quelconque des revendications 2, 3 ou 4 dans laquelle le domaine de liaison à l'héparine d'une autre superoxide dismutase qui comporte effectivement un domaine de liaison à l'héparine provient d'une superoxide dismutase extracellulaire.

6. Une superoxide dismutase selon la revendication 5 dans laquelle le domaine de liaison à l'héparine est un peptide présentant la séquence d'acides aminés suivante: 
Arg Gly Pro Gly Leu Trp Glu Arg Gln Ala Arg Glu His
Ser Glu Arg Lys Lys Arg Arg Arg Glu Ser Glu Cys Lys
Ala Ala
 ou un peptide présentant pratiquement la même séquence d'acides aminés et présentant pratiquement la même activité de liaison à l'héparine.

7. Une superoxide dismutase selon la revendication 5, dans laquelle le domaine de liaison à l'héparine est un peptide présentant la séquence d'acides aminés suivante : 
Cys Gly Pro Gly Ile Trp Glu Arg Gln Ala Arg Glu His
Ser Glu Arg Lys Lys Arg Arg Arg Glu Ser Glu Cys Lys
Ala Ala
 ou un peptide présentant pratiquement la même séquence d'acides aminés et ayant pratiquement la même activité de liaison à l'héparine.

8. Une superoxide dismutase selon l'une quelconque des revendications 2, 3 ou 4 dans laquelle le domaine de liaison à l'héparine de l'antithrombine III humaine est un peptide présentant la séquence d'acides aminés : 
Val Ala Lys Leu Asn Ser Arg Leu Tyr Arg Lys Ala Asn
Lys Ser Ser Lys Leu Val
 ou un peptide présentant pratiquement la même séquence d'acides aminés et présentant pratiquement la même activité de liaison à l'héparine.

9. Une superoxide dismutase selon l'une quelconque des revendications 2 à 8, dans laquelle la superoxide dismutase dépourvue du domaine de liaison à l'héparine est une superoxide dismutase érythrocytaire humaine.

10. ADN recombinant codant pour une superoxide dismutase selon l'une quelconque des revendications 2 à 9.

11. Un hôte transformé par un ADN recombinant selon la revendication 10 sous une forme susceptible de s'exprimer.

12. Un procédé de production d'une superoxide dismutase recombinante, comprenant la culture d'un hôte selon la revendication 11 pour produire la superoxide dismutase recombinante et l'isolement de la superoxide dismutase recombinante à partir de la culture.

13. Une préparation pharmaceutique comprenant une superoxide dismutase selon l'une quelconque des revendications 2 à 9.

14. Une préparation pharmaceutique selon la revendication 13 pour le traitement de l'oedème traumatique du cerveau, des troubles myocardiques ischémiques, de l'ulcère de l'estomac, de l'oedème hépatique ou des maladies ischémiques du foie.

15. L'utilisation d'une superoxide dismutase selon une des revendications 2 à 9 pour la préparation d'un médicament destiné au traitement de l'oedème traumatique du cerveau, des troubles myocardiques ischémiques, de l'ulcère de l'estomac, de l'oedème hépatique ou des maladies ischémiques du foie.
